(19) Europäisches Patentamt
European Patent Office
Office européen des brevets

(11) Publication number: **0 552 176 B1**

# (12) EUROPEAN PATENT SPECIFICATION

(45) Date of publication of patent specification: **02.11.95**

(51) Int. Cl.[6]: **C08L 5/08**

(21) Application number: **91916756.9**

(22) Date of filing: **16.08.91**

(86) International application number: **PCT/US91/05594**

(87) International publication number: **WO 92/06136 (16.04.92 92/09)**

The file contains technical information submitted after the application was filed and not included in this specification

## (54) OUATERNARY AMMONIUM SALTS DERIVED FROM CHITOSAN.

(30) Priority: **09.10.90 US 595121**

(43) Date of publication of application: **28.07.93 Bulletin 93/30**

(45) Publication of the grant of the patent: **02.11.95 Bulletin 95/44**

(84) Designated Contracting States: **AT BE CH DE DK ES FR GB GR IT LI LU NL SE**

(56) References cited:
**EP-A- 0 038 628**
**EP-A- 0 212 145**
**US-A- 4 071 478**
**US-A- 4 501 835**

**Bulletin of Chemical Society, volume 53, 1980, The Chemical Society of Japan, H. Fukuda: "Polyelectrolyte complexes of chitosan with sodium carboxymethylcellulose", pages 837-840 ;**

(73) Proprietor: **PFIZER INC.**
**235 East 42nd Street**
**New York, N.Y. 10017 (US)**

(72) Inventor: **CHANDLER, Charles, Edward**
**72 Church Hill Road**
**Ledyard, CT 06339 (US)**
Inventor: **CURATOLO, William, John**
**18 Patrick Place**
**Niantic, CT 06357 (US)**

(74) Representative: **Moore, James William, Dr. et al**
**Pfizer Limited**
**Ramsgate Road**
**Sandwich**
**Kent CT13 9NJ (GB)**

EP 0 552 176 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Die Makromolekulare Chemie, volume 175, 1974, Y. Kikuchi: "Polyelectrolyte complex of heparin with chitosan", pages 2209-2211 ;

## Description

This invention relates to quaternary ammonium salts of polysaccharides, the method of making, and the method of using the same, particularly as hypocholesterolemic agents.

### Background of the Invention

Generally, known products possessing hypocholesterolemic activity are cross-linked synthetic polymer derivatives, for example of polystyrene. These polymers are insoluble in water and thus have significant drawbacks. For example, cross-linked, water-insoluble, bile-acid-binding polystyrene-based resins, e.g., Cholestyramine®, have a gritty "mouth-feel", and thus have poor palatability. In addition, these resin beads have a low in vivo efficiency. Thus the effective hypocholesterolemic dose of these materials is excessive, typically 18-24 grams of formulated product per day.

Chitosan is a natural water soluble polymer that exhibits hypocholesterolemic activity when administered to a mammal in the form of a salt (e.g., the hydrochloride salt). However, in the known art (M. Sugano, T. Fujikawa et al., Am. J. of Clinical Nutrition, 33, April 1980, pp. 787, 793), the positive charges on the polysaccharide macromolecules of the chitosan are obtained by simply protonating the amino group, so that under the pH conditions of the intestinal tract (pH approximately 7.2), the proton is almost completely removed as the pKa of the chitosan is 6.3. For this reason the capacity of the polymer to interact with bile salts is very low.

U.S. Patent No. 4,436,731 teaches that when a chitosan quaternisation reaction is conducted directly on the amino group present in the amino-glucoside ring, the positive charge remains present under all pH and ionic conditions of the gastro-intestinal tract.

Published European Application 86108708.8, Publication No. 0212145, describes a variant of the compounds described in U.S. Patent No. 4,436,731 in which the positive charge is displaced from the polysaccharide backbone (in comparison to that disclosed in 4,436,731) on a branch which is easily available for interaction with the bile acid molecules. These quaternary ammonium salts of polysaccharides have the following general formula:

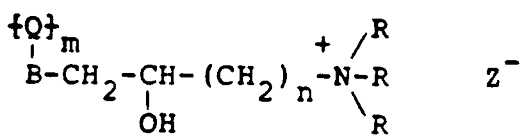

Formula I

in which Q represents the monomer unit of a natural polysaccharide, m is a whole number between 100 and 1000, n is a whole number between 0 and 4, B is 0 or S or NH or NR, R is a linear alkyl radical of 1-4 carbon atoms, and $Z^-$ is an anion such as $Cl^-$, $Br^-$, $I^-$, $HSO_4^-$, or $CH_3O\text{-}SO_3^-$.

In the compounds of U.S. 4,436,731 and EP 0,212,145, the polycationic polysaccharide has an anionic counterion (e.g., $Z^-$ in the formula I compounds). Typically, this counterion is an inorganic, or low molecular weight organic, radical having a single negative charge (e.g., $Cl^-$ or $CH_3SO_3^-$).

Many of the hypocholesterolemic compounds of U.S. 4,436,731 and EP 0,212,145 are water soluble. As such they have greater palatability than the water insoluble compounds of the cholestyramine type.

Finally U.S. Patent No. 4,436,731 teaches the use of gum arabic as a pharmaceutical carrier for a polycationic polysaccharide. However, there is no mention or suggestion of salt formation with this letter species.

U.S. Patent No. 4,071,478 does teach the use of alginate and polyacrylate as counterions to nonpolysaccharide polycation bile acid sequestrants.

Although the above compounds make a significant contribution to the art there is a continuing search in this field of art for improved hypocholesterolemic pharmaceuticals.

Summary of the Invention

It has been discovered that the above water soluble polycationic polysaccharides suffer from side effects. They show cytotoxicity, and they have been observed to cause substantial gastrointestinal side effects.

The present invention is based on the discovery that these unwanted properties of the water soluble, polycationic bile acid sequestrants can be greatly reduced or eliminated by forming a salt of the polycation with a counterion ($X^{n-}$) which has a plurality of negative charges on each molecule. This discovery is particularly applicable to the compounds of formula I above, in which Q is derived from chitosan, and $X^{n-}$ is gum arabic or carboxymethyl cellulose.

Thus this invention provides new quaternary ammonium salts of chitosan possessing hypocholesterolemic activity of the formula

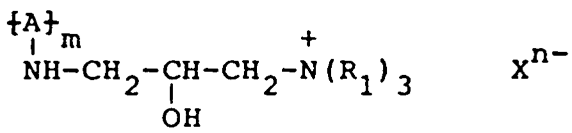

Formula II

in which A represents the monomer unit of a chitosan, m is a whole number between 100 and 1,000, $R_1$ is $C_1$-$C_4$ linear alkyl and $X^{n-}$ is a physiologically acceptable water soluble polyanion, where n is at least 10 but not greater than 10,000, and any remaining positive charges are balanced by nonpolymeric anions,wherein said polyanion is gum arabic or carboxymethyl cellulose and the proportion of polycation to polyanion $X^{n-}$ is from 1 to 5,to 20 to 1 by weight. Nonpolymeric anions are anions having negative charges less than 10 such as inorganic or organic anions (e.g., chloride, bromide, iodide, sulfate, benzoate, succinate or glucuronate).

Preferred salts of the invention are the salts of formula II, wherein the alkyl substituent is methyl.

Another aspect of this invention is a process for preparing quaternary ammonium salts of chitosan possessing hypocholesterolemic activity by contacting a compound of the formula:

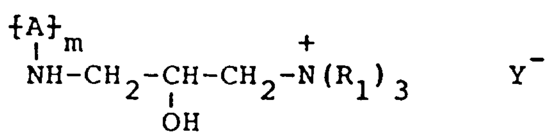

Formula III

in which A represents the monomer unit of a chitosan, m is a whole number between 100 and 1,000, $R_1$ is $C_1$-$C_4$ linear alkyl and Y- is a physiologically acceptable water soluble inorganic or monomeric organic counterion, with gum arabic or carboxymethylcellulose.

The present invention is also directed to pharmaceutical compositions for the control of hypercholesterolemia which comprise a salt of claim 1 in a pharmaceutically acceptable carrier.

Another aspect of this invention is a process for preparing a pharmaceutical composition of claim 1 comprising dissolving a compound of formula III and gum arabic or carboxymethylcellulose in a common protic solvent, precipitating the thus formed polycation-polyanion salt, collecting the polycation-polyanion salt and adding sufficient inorganic salt such that said polycation-polyanion salt is substantially soluble upon mixing in an aqueous solution.

Other features and advantages will be apparent from the specification and claims which describe an embodiment of this invention.

Detailed Description of the Invention

The compounds of this invention may be made by contacting a compound of formula III and a physiologically acceptable water soluble polyanion. Thus the two components may be combined in a solution in order to form the salt. However, the two polymeric polyions may also be combined in the solid form as a mixture and later solubilized (e.g., in vivo) to form the formula III compound. Exemplary physiologically acceptable inorganic or monomeric organic counterions of Formula III are $Br^-$, $Cl^-$, $HSO_4^-$, lactate$^-$ and benzoate$^-$.

Any solvent (or combination of solvents) may be used that solubilizes the polycation and polyanion so that they form the desired compound of formula II. Generally any protic solvent performs the above function. Preferably an aqueous solution is used as it is nontoxic. The components are conveniently combined at ambient conditions. However, the components may also for example, be combined at a temperature of about 0°C to about 100°C. Any reduced pressure at which the solvent does not boil and elevated pressures up to 10 atm. and above may be used. The salt formation may occur instantaneously, or may require mixing until both components are dissolved and well mixed (typically within 2 days).

The proportion of Formula III polycation to polyanion is from 1 to 5 to 20 to 1, because below about 1 to 5 the absolute dose becomes too large to be practical for human therapy and above about 20 to 1 the protection afforded by the polyanion becomes relatively insignificant. It is preferred that the proportion of formula III compound to polyanion is about 1 to 2 to about 5 to 1. The resultant complex (polycation-polyanion) typically falls within the above proportions.

A desired form of the salt is a water soluble powder. This may be produced by combining a compound of formula III and the desired polyanion in a common protic solvent in order to form a polycation-polyanion salt which is then precipitated. The order of addition of a formula III compound solution and an anionic polymer solution is not critical (See Example 7). The precipitation can be performed by a variety of procedures such as spray drying, reduction in ionic strength, change in pH, addition of a non-solvent and freeze-drying. The ionic strength may be changed by reducing the concentration of components (See Examples 8, 12, 13). The precipitate is collected and sufficient water soluble physiologically acceptable salts are added to the mixture to ensure solubility upon redissolution (See Examples 9, 11 and 12). Typically inorganic salts such as sodium chloride, sodium phosphate, and potassium chloride may be used, however, organic salts such as sodium or potassium benzoate, ascorbate, citrate, or acetate may also be used.

Surprisingly a solution of the polycation-polyanion salt can be precipitated by, for example, further dilution of the salt. Upon addition of additional water soluble salts, the precipitate can be redissolved. Thus, this provides a simple method for collecting the polycation-polyanion salt and producing a water soluble product from the precipitate by addition of sufficient quantities of a suitable salt.

The compound of formula III may be produced by reacting chitosan (typically pretreated as described below) with a salt having formula IV in a reaction medium consisting of a mixed solvent as described below.

Chitosan +

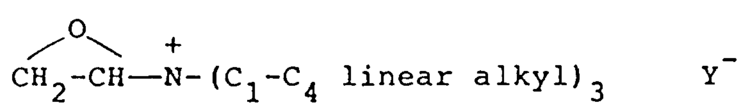

Formula IV

The resulting quaternary ammonium salts of chitosan preferably have a degree of substitution of between 0.5 and 2. The term "degree of substitution" signifies the ratio of the number of moles of side chain containing the quaternary ammonium group to the number of monomer units of the chitosan.

The chitosan to be reacted with the compound of formula IV may be subjected to pretreatment with an acid, with a base or with a solvent in order to adjust its molecular weight to within the desired range and to increase its reactivity. Water-insoluble chitosan is either pretreated by acid hydrolysis to obtain polymers with a suitable molecular weight of between 10,000 and 300,000 or are dissolved in suitable solvents (for example formic acid) and are then precipitated in an aqueous environment at pH 10-12 to obtain an almost amorphous polymer able to also react in the heterogenous phase.

The reaction between the chitosan and compound of formula IV is conducted in a solvent under agitation at a temperature of between 40°C and 100°C for a time of between 4 and 16 hours with a molar ratio of chitosan to epoxy compound of between 1:1 and 1:6 and a weight ratio of chitosan to reaction

medium of between 1:5 and 1:35.

The compounds of this invention are all readily adapted to therapeutic use as hypocholesterolemic agents. They are administered orally in dosages ranging from 60 to 1000 mg/kg of body weight per day in single or divided doses. Of course, in particular situations, at the discretion of the attending physician, doses outside of this range will be used.

The compounds of this invention can be administered in a wide variety of different dosage forms, i.e., they may be combined with various pharmaceutically-acceptable inert carriers in the form of tablets, capsules, lozenges, troches, hard and soft candies, powders, elixirs, or syrups and the like. Such carriers include solid diluents or fillers, aqueous media and various non-toxic organic solvents.

For purposes of oral administration, tablets containing various excipients may be employed. For example sodium citrate, calcium carbonate and calcium phosphate may be employed along with various disintegrants such as starch and preferably potato or tapioca starch, alginic acid and certain complex silicates, together with binding agents such as polyvinyl pyrrolidone, sucrose, gelatin and acacia. Additionally, lubricating agents such as magnesium stearate, sodium lauryl sulfate and talc are often very useful for tableting purposes. Solid compositions of a similar type are also employed as fillers in soft and hard gelatin capsules; preferred materials in this connection also include lactose or milk sugar as well as high molecular weight polyethylene glycols. When aqueous suspensions and/or elixirs are desired for oral administration, the essential active ingredient therein can be combined with various sweetening agents, flavoring agents, coloring agents, emulsifying agents and/or suspending agents, as well as such diluents as water, ethanol, propylene glycol, glycerin and various like combinations thereof.

In a pharmaceutical composition comprising a compound of formula II, the weight ratio of carrier to active ingredient will normally be in the range from 1 to 19 to 9 to 1. However, in any given case, the ratio chosen will depend on such factors as the solubility of the active component, the dosage contemplated and the precise route of administration.

The compounds of this invention are useful for the control of hypercholesterolemia. The activity can be determined by, for example, the dosage (by oral gavage) which reduces serum blood cholesterol of male Golden Syrian hamsters. The hamsters received doses of the polycation-polyanion once a day for 5 days and approximately one hour after the compound dosage on day 5, the hamsters were sacrificed and serum samples taken. Solutions of the formula III compound in 1% and 2% gum arabic reduced the cholesterol levels and protected the gastrointestinal tract of animals from damage observed when gum arabic was used at lower concentrations. The details and results of these experiments are further detailed in Examples 1 and 2 below.

Activity can also be determined by for example the amount of polyanion that reduces the cytotoxic/cytostatic effects associated with formula III compounds on mouse lymphoma or mouse mastocytoma cells.

Mouse lymphoma cells grown in suspension culture were treated with the polycation-polyanion for 3 hours and subsequent cell growth was measured for an additional 45 hours. Cytostatic effects on mouse lymphoma cells associated with 5 micro-g/ml - 320 micro-g/ml concentrations of formula III compounds were reduced by the inclusion of carboxymethylcellulose in the culture medium at concentrations nearly equal to or greater than the concentration of the formula III compound tested. The details and results of these experiments are further detailed in Example 3 below.

Mouse mastocytoma cells grown in suspension culture were treated with the polycation-polyanion for 3 hours and resultant cytotoxicity was assessed by release of previously incorporated radiolabeled $^{51}$CR into the culture medium. Cytotoxic effects of formula III compounds on the mouse mastocytoma cells were reduced by the inclusion of carboxymethyl-cellulose, polyacrylic acid, carbopol, or gum arabic. The details and results of these experiments are further detailed in Example 4 below.

Activity can also be determined by for example the amount of polyanion that reduces a decrease in initial cell adhesion associated with formula III compound effects on HLLC18 clone of HEP-G2 cells. HLLC18 cells were treated for 3 hours with the polycation-polyanion during the initial attachment phase of cell growth after having been enzymatically removed from tissue culture flasks. Formula III compound was associated with a decrease in initial plating efficiency of the HLLC18 clone of HEP-G2 cells which was prevented by the inclusion of 50 micro-g/ml carboxymethylcellulose. The details and results of these experiments are further detailed in Example 5 below.

EXAMPLE 1

Serum cholesterol lowering in chow-fed Golden Syrian hamsters dosed by oral gavage with 10% (w/v) a compound of Formula III-A (herein defined as a Formula III compound wherein $Y^-$ is $Cl^-$ and $R_1$ is methyl)

(1 g/kg) dissolved in solutions of .25% (w/v) gum arabic (25 mg/kg), .5% (w/v) gum arabic (50 mg/kg), 1.0% (w/v) gum arabic (100 mg/kg), or 2.0% (w/v) gum arabic (200 mg/kg).

Six groups of 5 or 6 male Golden Syrian hamsters were acclimated to Agway RMH rodent chow and reversed light cycle (lights on 3 p.m. to 3 a.m.) for approximately one week prior to initiation of the following experiment. Hamsters mean body weight at beginning of experiment was approximately 144 g. On days 1, 2, 3, 4 and 5 test compounds were presented to the animals by oral gavage of 1 ml test solution per 100 g body weight between 9 a.m. and 10 a.m. Group 1 received water. Group 2 received 2% (w/v) gum arabic (200 mg/kg). Group 3 received 10% (w/v) a compound of Formula III-A (1 g/kg) dissolved in .25% (w/v) gum arabic (25 mg/kg). Group 4 received 10% (w/v) a compound of Formula III-A (1 g/kg) dissolved in .50% (w/v) gum arabic (50 mg/kg). Group 5 received 10% (w/v) a compound of Formula III-A (1 g/kg) dissolved in 1% (w/v) gum arabic (100 mg/kg). Group 6 received 10% (w/v) a compound of Formula III-A (1 g/kg) dissolved in 2% (w/v) gum arabic (200 mg/kg). All of these solutions were clear and free from precipitated material. Hamsters were allowed free access to pelleted rodent chow and water throughout the experiment. Approximately 1 hour after the compound dose on day 5, the hamsters were sacrificed under pentobarbital anesthesia and blood was collected via cardiac puncture. Serum obtained from the blood samples was analyzed for total cholesterol.

Results: Group 1 - 142 ± 10 mg/dl; Group 2 - 133 ± 17 mg/dl; Group 3 - 120 ± 39 mg/dl; Group 4 - 129 ± 27 mg/dl; Group 5 - 117 ± 17 mg/dl; Group 6 - 110 ± 8 mg/dl. GI tracts of animals in Groups 1, 2, 5, and 6 looked normal. In Group 3, 4 of the 5 hamsters developed diarrhea, and upon inspection at sacrifice, one had enlarged seminal vesicles, all had small gall bladders and small livers. In Group 4, 3 of 5 hamsters developed diarrhea, and upon inspection at sacrifice two had bloodly cecums, one had internal organ adhesions.

## EXAMPLE 2

Serum cholesterol lowering in chow-fed Golden Syrian hamsters dosed by oral gavage with 10% (w/v) a compound of Formula III-A (1 g/kg) dissolved in solution of 10% (w/v) gum arabic (1 g/kg) or with 10% (w/v) a compound of Formula III-A (1 g/kg) dissolved in solution of 2% (w/v) gum arabic (200 mg/kg).

Four groups of 5 (or 3 in Group 4) male Golden Syrian hamsters were acclimated to Agway RMH rodent chow and reversed light cycle (lights on 3 p.m. to 3 a.m.) for approximately one week prior to initiation of the following experiment. Hamsters mean body weight at beginning of experiment was approximately 136 g. On days 1, 2, and 3 test compounds were presented to the animals by oral gavage of 1 ml test solution per 100 g body weight between 9 a.m. and 10 a.m. Group 1 received water; Group 2 received 10% (w/v) gum arabic (1 g/kg); Group 3 received 10% (w/v) a compound of Formula III-A (1 g/kg) dissolved in 10% (w/v) gum arabic (1 g/kg). Group 4 received 10% (w/v) a compound of Formula III-A (1 g/kg) dissolved in 2% (w/v) gum arabic (200 mg/kg). All of these solutions were clear and free from precipitated material. Hamsters were allowed free access to pelleted rodent chow and water throughout the experiment. Approximately 1 hour after the compound dose on day 3, the hamsters were sacrificed under pentobarbital anesthesia and blood was collected via cardiac puncture. Serum obtained from the blood samples was analyzed for total cholesterol.

Results: Group 1 - 108 ± 8 mg/dl; Group 2 - 109 ± 11 mg/dl; Group 3 - 88 ± 12 mg/dl; Group 4 90 ± 17 mg/dl. GI tracts of animals in all groups looked normal.

## EXAMPLE 3

Cell cytotoxicity caused by a compound of Formula III-A can be prevented by carboxymethylcellulose.

A stock solution of a compound of Formula III-A was made in sterile deionized water at 64 mg/ml by dissolving 115.9 mg in 1.81 ml. This was diluted serially in sterile deionized water to give additional stock solutions of 16 mg/ml, 4 mg/ml, and 1 mg/ml. A stock solution of carboxymethylcellulose was made at 10 mg/ml in sterile deionized water by dissolving 23.92 mg of carboxymethylcellulose in 2.39 ml. This was further diluted in sterile deionized water to another stock solution of 2 mg/ml. Fifteen tubes were set up such that #1-#5 received 100 micro-l of sterile deionized water, #6-#10 received 100 micro-l of the 2 mg/ml stock solution of carboxymethylcellulose, and #11-#15 received 100 micro-l of the 10 mg/ml stock solution of carboxymethylcellulose. Further, tubes #1, #6, and #11 received 100 micro-l of sterile, deionized water; tubes #2, #7, and #12 received 100 micro-l of the 1 mg/ml stock solution of Formula III-A; tubes #3, #8, and #13 received 100 micro-l of the 4 mg/ml stock solution of a compound of Formula III-A; tubes #4, #9, and #13 received 100 micro-l of the 16 mg/ml stock solution of a compound of Formula III-A; and tubes #5, #10, and #15 received 100 micro-l of the 64 mg/ml stock solution of a compound of Formula III-A. These mixes

gave three series of 5 tubes that contained 0, 500, 2000, 8000, and 32,000 micro-g/ml of a compound of Formula III-A in water, in 1000 micro-g/ml carboxymethylcellulose, or in 5000 micro-g/ml carboxymethylcellulose. In the series made in the 1000 micro-g/ml carboxymethylcellulose, all tubes containing a compound of Formula III-A showed some turbidity. In the series made in the 5000 micro-g/ml carboxymethylcellulose, the solutions containing 0 micro-g/ml and 500 micro-g/ml remained transparent and the rest showed turbidity.

Mouse lymphoma cells (L5178Y 3.7.2C/TK±) were grown in RPMI 1640 tissue culture medium containing 10% (v/v) fetal bovine serum. 10 ml of cell suspension (6 x 10E5/ml) was added to each of 15 sterile 50 ml polypropylene culture tubes that were numbered from #1 to #15. 100 micro-l of the correspondingly numbered solutions described above above was added to each of these cell suspensions. This diluted the concentrations of both the compound of Formula III-A and the carboxymethylcellulose by a factor of 100. The capped tubes containing the cell suspensions were placed in rotating drums in a 37°C incubator for 3 hours. The tubes were removed from the incubator and were pelleted by centrifugation. The medium was decanted and replaced by 10 ml of fresh growth medium. After another centrifugation, this step was repeated once. The final cell pellet was then suspended in 20 ml of growth medium such that the cell concentration was 3 x 10E5 cells/ml. The tubes were returned to the incubator.

Eighteen hours and 24 hours later, 1 ml of cell suspension from each tube was added to 9 ml of counting solution (Isoton II [TM]; Coulter, Inc.). The cell concentration was then determined using a Coulter cell counter. From the data obtained at the 24 hour time point, the cell concentration in each tube was diluted with fresh growth medium to a final cell concentration of 2 x 10E5 cells/ml before being put back into the incubator for an additional 24 hours. At this time, cell concentrations were determined as just described.

Results: Tubes #1-#5 (no carboxymethylcellulose; 0, 5, 20, 80 320 micro-g/ml a compound of Formula III-A) had final calculated cell concentrations (determined by the product of the dilution at 24 hours and the final cell concentration reached at 48 hours) of 36.8, 15.91, 2.94, 5.52, and 2.22 x 10E5 cells/ml respectively. Tubes #6-#10 (10 micro-g/ml carboxymethylcellulose; 0, 5, 20, 80, 320 micro-g/ml a compound of Formula III-A) had final calculated cell concentration of 47.23, 46.40, 19.35, 3.30, and 2.55 x 10E5 cells/ml respectively. Tubes #6-#10 (50 micro-g/ml carboxymethylcellulose; 0, 5, 20, 80 320 micro-g/ml a compound of Formula III-A) had final calculated cell concentrations of 39.19, 47.5, 48.79, 4.38, and 1.42 x 10E5 cells/ml respectively.

EXAMPLE 4

Carboxymethylcellulose, polyacrylic acid (2000 $M_r$), carbopol (polyacrylic acid co-polymer with polyalkyl sucrose) and gum arabic protect mouse mastocytoma cells from polycationic-induced cytotoxicity.

Mouse mastocytoma cells (P815) were grown in suspension culture in RPMI 1640 medium plus 10% fetal bovine serum (FBS) using standard tissue culture techniques. On the day of the assay, 5 x 10E6 cells were suspended in 100 micro-l of Hank's Balanced Salt Solution (HBSS) plus 10% FBS. 50 micro-l of $^{51}$Cr was added to the cells and they were incubated in a shaking water bath at 37°C for 30 minutes during which time they took up the $^{51}$Cr. The cells were then washed 4 times in 15 ml HBSS plus 10% FBS. They were resuspended in RPMI 1640 medium plus 10% FBS at approximately 2.5 x 10E5/ml.

To begin the cytotoxicity assay, 100 micro-l of effector solution (e.g., test agent plus polyanionic polymers; see below) at 1.2 x final concentration was added to quadruplicate wells of V-bottomed microtiter plates. To being the assay, 20 micro-l of $^{51}$Cr-loaded mouse mastocytoma cells were added to each well resulting in approximately 5000 cells per well. The plate was then incubated at 37°C in a tissue culture incubator. The remaining effector solution was vortexed and 150 micro-l of each was pipetted into 3 wells each of flat-bottomed microtiter plates and the optical density at 610 nm read with a plate reader as a measure of solution turbidity.

After the 3 hour incubation, the microtiter plates were centrifuged to pellet any suspended cells and 50 micro-l samples of medium from each well were counted for radioactivity. Radioactivity released from cells incubated with 0.1% Triton X-100 (a detergent) was used as the 100% control. Medium from cells incubated withou effector substanced was used to measure spontaneous $^{51}$Cr release.

$$\% \ ^{51}\text{Cr released} = (\text{sample dpm - spontaneous dpm})/(\text{TX-100 dpm - spontaneous dpm}).$$

Stock solutions of a compound of Formula III-A, carboxymethylcellulose-LF, carbopol, gum arabic, and polyacrylic acid were all made in sterile water at 3% (w/v; 30,000 micro-g/ml). A compound of Formula III-A was diluted to 303.03 micro-g/ml and 612.24 micro-g/ml with RPMI 1640 plus 10% FBS. For one series

(final concentration of a compound of Formula III-A = 250 micro-g/ml) 990 micro-l of the 303.03 micro-g/ml stock was mixed with 10 micro-l of polyanion stock solution (see below) and 100 micro-l of this was added to each of quadruplicate wells. For the other series (final concentration of a compound of Formula III-A = 500 micro-g/ml) 980 micro-l of the 612.24 micro-g/ml stock was mixed with 20 micro-l of polyanion stock solution (see below) and 100 micro-l was added to each of quaduplicate wells.

This experiment was designed to test two concentrations of a compound of Formula III-A (250 micro-g/ml and 500 micro-g/ml; final concentration) and to titrate the various polyanionic compounds so that they comprised from 0% to 50% of the total polymeric compounds in the assay. Thus, 50% anionic polymer is a 1:1 mix of a compound of Formula III-A and the test anionic polymer. These mixes were accomplished by diluting each of the polyanionic polymer stock solutions (3% w/v; 30,000 micro-g/ml) with sterile water to form solutions of 2.45%, 2%, 1.61%, 1.29%, 1%, 0.75%, 0.53%, 0.33% and 0.16% (w/v) so that by mixing 10 micro-l (for a compound of Formula III-A at 250 micro-g/ml series) or 20 micro-l (for a compound of Formula III-A at 500 micro-g/ml series) one obtains mixtures at 5% intervals from 0% polyanion to 50% polyanion. For example, 20 micro-l of the 0.53% polyanion mixed with 980 micro-l of 612.24 micro-g/ml a compound of Formula III-A gives a solution that has 106 micro-g/ml polyanion and 600 micro-g/ml a compound of Formula III-A. The polyanion makes up 15% of the total polymer in the solution in this example.

Results: Carboxymethylcellulose: A compound of Formula III-A at 0% CMC caused ~35% and ~40% release of $^{51}$Cr from the cells at 250 micro-g/ml and 500 mg/ml respectively. This decreased to background in both series by 35% CMC. Concommitantly, the medium turbidity increased as the percent polymer increased in both series.

Carbapol: A compound of Formula III-A at 0% Carbapol caused ~32% and ~58% release of $^{51}$Cr from the cells at 250 mg/ml and 500 mg/ml respectively. This decreased to near background in both series by 40% Carbapol. Concommitantly, the medium turbidity increased as the percent polymer increased in both series.

Polyacrylic acid: A compound of Formula III-A at 0% polyacrylic acid caused ~30% and ~60% release of $^{51}$Cr from the cells at 250 mg/ml and 500 mg/ml respectively. This decreased to near background in both series by 30% polyacrylic acid. Concommitantly, the medium turbidity increased as the percent polymer increased in both series.

Gum Arabic: A compound of Formula III-A at 0% gum arabic caused ~34% and ~42% release of $^{51}$Cr from the cells at 250 mg/ml and 500 mg/ml respectively. This decreased in both series with increasing percentage of gum arabic. Concommitantly, the medium turbidity increased slightly as the percent polymer increased in both series.

## EXAMPLE 5

A compound of Formula III-A causes a decrease in initial cell adhesion to tissue culture plastic that can be prevented by inclusion of carboxymethylcellulose.

HEP-G2 cells (clone HLLC18) were grown in 175 $cm^2$ tissue culture flasks using standard techniques. The cells were metabolically labelled with 60 micro-Ci$^3$H-glycine for 20 hours. The cell monolayer was washed 3 times with fresh medium to remove non-incorporated radiolabel. Cells were enzymatically removed from the flask and suspended at 2 x 10E5 cells/ml in complete medium.

A stock solution of 1000 micro-g/ml a compound of Formula III-A was made in tissue culture medium by dissolving 5 mg compound of Formula III-A in 5 ml of tissue culture medium. This stock solution was further diluted in tissue culture medium to give 2 ml of working stock solutions of a compound of Formula III-A at 400, 300, 200, 100, 40, and 20 micro-g/ml. A 2000 micro-g/ml stock solution of carboxymethylcellulose was made in tissue culture medium by dissolving 20 mg of carboxymethylcellulose in 10 ml medium. This was diluted by a factor of 10 in tissue culture medium to give a working stock solution of 200 micro-g/ml.

To each well of a 24 well tissue culture plate, 250 micro-l of medium or 250 micro-l of the 200 micro-g/ml working stock of carboxymethylcellulose was added. Then, to each well, 250 microliters of each medium or 250 microliters of the various stock solutions of a compound of Fomula II-A compound was added. Finally, to each well, 500 micro-l of the above cell suspension was added, the plate was gently swirled and placed in a humidified tissue culture incubator containing 95% air/5% $CO_2$ at 37 °C for 3 hours. At the end of the incubation, the plates were swirled 10 times, tipped at a 45° angle, and the medium was aspirated. Attached cells were dissolved by adding 1 ml of 0.1 N NaOH. 800 micro-l of this was transferred to a scintillation vial, 200 micro-l of 4N HCl was added and the vials counted for radioactivity. This radioactivity was compared to that obtained from 80% of the number of cells that had initially been plated.

Thus the conditions tested consisted of 2 series: one in which the effects of 0, 5, 10, 25, 50, 75, 100, and 250 micro-g/ml of a compound of Formula III-A were examined; and the second in which the effects of the same concentrations of a compound of Formula III-A plus 50 micro-g/ml carboxymethylcellulose were examined.

Results: A compound of Formula III-A decreased initial plating efficiency in a dose dependent manner as follows (0 micro-g/ml to 250 micro-g/ml as described above): 86%, 82%, 86%, 86%, 83%, 74%, 76%, 67%. The decrease was significant at compound Formula III-A concentrations equal to and greater than 75 micro-g/ml. In the presence of 50 micro-g/ml carboxymethylcellulose and the same compound Formula III-A concentrations as above, the plating efficiencies were: 85%, 84%, 85%, 85%, 75%, 80%, 82%, and 83%.

EXAMPLE 6

Physical Characteristics of Solutions of Cationic Polymers Formula III compound when mixed with Solutions of Gum Arabic.

A stock solution of a Formula III-A compound was made in water at 50 mg/ml (5% w/v) by dissolving 190 mg of a formula III-A compound in 3.8 ml deionized water. The solution was transparent. A stock solution of gum arabic (an example of an anionic polymer) was made in water at 50 mg/ml (5% w/v) by dissolving 413 mg of gum arabic in 8.27 ml of deionized water. This solution was transparent. 50 micro-l of water was pipetted into 6 small glass vials and 50 micro-l of a formula III-A compound was pipetted into 6 additional vials. This gave 2 series of vials containing water and formula III-A compound. Vial 1 of each series then received 150 micro-l water, vial 2 133.3 micro-l water, vial 3 125 micro-l water, vial 4 100 micro-l water', vial 5 50 micro-l water, and vial 6 no addition. Then no addition was made to vial 1, 16.67 micro-l gum arabic was added to vial 2, 25 micro-l of gum arabic to vial 3, 50 micro-l gum arabic to vial 4, 100 micro-l gum arabic to vial 5, and 150 micro-l gum arabic to vial 6. One additional vial received only 200 micro-l of gum arabic. Thus two series of 6 vials were obtained that contained from 0 to 37.5 mg/ml gum arabic alone or 0 to 37.5 mg/ml gum arabic + 12.5 mg/ml a compound of Formula III-A. The last vial contained only 50 mg/ml gum arabic. All vials were mixed thoroughly by vortexing and were then observed.

Results: All the vials containing gum arabic alone remained transparent. The vials with no gum arabic and 4.17 mg/ml gum arabic (3:1::a compound of Formula III-A:GA) remained transparent. The vial containing gum arabic at 6.25 mg/ml (2:1::a compound of Formula III-A:GA) was slightly cloudy, the vial containing gum arabic at 12.5 mg/ml (1:1::a compound of Formula III-A:GA) was cloudy, and the vials containing 25 mg/ml and 37.5 mg/ml gum arabic (1:2 and 1:3::a compound of Formula III-A:GA) demonstrated frank precipitation.

EXAMPLE 7

Order of addition of cationic polymer solution or anionic polymer solution is not important to the formation of a precipitate.

Stock solutions were same as used in Example 6. 100 micro-l of gum arabic was added to vial 1 and 100 micro-l of a compound of Formula III-A was added to vial 2. 100 micro-l of a compound of Formula III-A was then added to vial 1 and 100 micro-l gum arabic was added to vial 2. Vials were mixed and then observed.

Results: Both vials turned cloudy.

EXAMPLE 8

Solubility of 1:1 mixes of a compound of Formula III-A and gum arabic in water are dependent upon polymer concentration.

A 10% (w/v) solution of gum arabic was made in water by dissolving 10 g of gum arabic into a final volume of 100 ml of deionized water. A 10% (w/v) solution of a compound of Formula III-A was made in the 10% (w/v) solution of gum arabic by dissolving 5 g of a compound of Formula III-A in a final volume of 50 ml. The resulting solution was transparent. 200 micro-l of this solution was mixed with 200 micro-l deionized water and the resulting solution (5% w/v in each polymer) was transparent. The addition of 50 micro-l more deionized water (4.44% w/v each polymer) started to turn cloudy. An additional 50 micro-l of deionized water (4.0% w/v in each polymer) was added and the solution turned cloudy.

EXAMPLE 9

Solubility of 1:1 mixes of a compound of Formula III-A and gum arabic in water are dependent upon concentration and ionic strength.

A 10% (w/v) solution of gum arabic was made in water by dissolving 10 g of gum arabic into a final volume of 100 ml of deionized water. A 10% (w/v) solution of a compound of Formula III-A was made in the 10% (w/v) solution of gun arabic by dissolving 5 g of a compound of Formula III-A in a final volume of 50 ml. The resulting solution was transparent. 200 micro-l of this solution was mixed with 200 micro-l 154 mM NaCl and the resulting solution (5% w/v in each polymer; 77 mM NaCl) was transparent. The solution after addition of 200 micro-l more 154 mM NaCl (3.33% w/v each polymer; 103 mM NaCl) remained transparent. An additional 200 micro-l of 154 mM NaCl (2.5% w/v in each polymer; 116 mM NaCl) turned the solution slightly cloudy. An additional 200 micro-l of 154 mM NaCl (2.0% w/v each polymer; 123 mM NaCl) also gave a slightly cloudy solution. Additional 154 mM NaCl (200 micro-l and 400 micro-l bringing each polymer to 1.67% and 1.43% w/v and NaCl to 128 mM and 132 mM respectively) gave resultant solutions that were turbid.

EXAMPLE 10

Decreased pH brings precipitated compound of Formula III-A gum arabic precipitate back into solution.

A 10% (w/v) solution of gum arabic was made in water by dissolving 10 g of gum arabic into a final volume of 100 ml of deionized water. A 10% (w/v) solution of a compound of Formula III-A was made in the 10% (w/v) solution of gum arabic by dissolving 5 g of a compound of Formula III-A in a final volume of 50 ml. The resulting solution was transparent. One ml of the above solution was mixed with 3 ml of deionized water and the solution turned turbid. Three 1 ml aliquots of the turbid solution were mixed with 10 micro-l of 1N NaOH, 1N HCl, or 1M NaCl (resulting in final concentration of 10 mM for the base, acid, or salt). Only the sample mixed with HCl went back into a transparent solution. pH of this solution was approximately 3 (as measured by pH test strips).

EXAMPLE 11

1:1 mixes of a compound of Formula III-A and gum arabic remain soluble in solutions of increased ionic strengths.

A 10% (w/v) solution of gum arabic was made in water by dissolving 10 g of gum arabic into a final volume of 100 ml of deionized water. A 10% (w/v) solution of a compound of Formula III-A was made in the 10% (w/v) solution of gum arabic by dissolving 5 g of a compound of Formula III-A in a final volume of 50 ml. The resulting solution was transparent. 500 mM Na phosphate buffer, pH 7.1 was prepared by mixing 500 mM solutions of dibasic (4 ml) and monobasic (1 ml) Na phosphate together. This Na phosphate solution was further diluted with water to give concentrations of 400 mM, 300 mM, 200 mM, 100 mM, 80 mM, 60 mM, 40 mM, and 20 mM. 750 micro-l of each of these was mixed with 250 micro-l of the 10% (w/v) Formula III-A in 10% (w/v) of a compound of gum arabic so that the resulting solutions were 2.5% (w/v) in each polymer and 375 mM, 300mM, 225 mM, 150 mM, 75 mM, 60 mM, 45 mM, 30 mM, and 15 mM in Na phosphate.

Results: At Na phosphate concentrations greater than 75 mM, the resulting solutions were transparent. At Na phosphate concentrations of 75 mM and 60 mM the solutions were slightly cloudy, and at all lower Na phosphate concentrations, the solutions were very cloudy.

EXAMPLE 12

Increased ionic strength allows 1:1 mixes of a compound of Formula III-A and carboxymethylcellulose to remain soluble upon dilution.

Stock solutions of a compound of Formula III-A and carboxymethylcellulose were made in water at 50 mg/ml (5% w/v) by dissolving 190 mg of a compound of Formula III-A in 3.8 ml deionized water or 353 mg of carboxymethylcellulose in 7.06 ml deionized water. These solutions were transparent. Each of these stock solutions were further diluted to a final concentration of 2.5% (w/v) by mixing 500 micro-l of the stock with either 500 micro-l deionized water or 500 micro-l 500 mM Na phosphate buffer, pH 7.1. This resulted in 2 solutions of each polymer (2.5% w/v) either in deionized water or 250 mM Na phosphate: 100 micro-l of each solution of a compound of Formula III-A was mixed with 100 micro-l of either carboxymethylcellulose made in deionized water or in Na phosphate buffer. All four mixes contained 1.25% (w/v) of each polymer,

and either 0 mM, 125 mM (two tubes), or 250 mM Na phosphate.

Results: The tube at 0 mM Na phosphate gave a precipitate, the tubes at 125 mM Na phosphate were cloudy, while the tube at 250 mM Na phosphate was very slightly cloudy.

EXAMPLE 13

Stock solutions of a compound of Formula III-A and gum arabic were made at 100 mg/ml (10% w/v) in water by dissolving 5 g of each in a final volume of 50 ml dionized water. 10 ml of each of these were mixed together with a resultant clear solution (5% w/v in each polymer). Upon the addition of 30 ml of deionized water, a precipitate formed (2% w/v of each polymer). This tube was centrifuged at 15,000 x g for 15 minutes and approximately 2 ml of precipitate was pelleted. In another experiment, 10 ml of 10% (w/v) of a compound of Formula III-A and 20 ml of 10% (w/v) gum arabic were mixed together and a turbid solution resulted (3.33% w/v of a compound of Formula III-A and 6.66% w/v gum arabic). 20 ml of deionized water was added and the solution remained turbid (2% w/v of a compound of Formula III-A and 4% gum arabic). This tube was centrifuged at 15,000 x g for 15 minutes and approximately 7.5 ml of precipitate was pelleted.

**Claims**

**Claims for the following Contracting States : AT, BE, CH, DE, DK, FR, GB, IT, LI, LU, NL, SE**

**1.** Quaternary ammonium salts of chitosan possessing hypocholesterolemic activity of the formula:

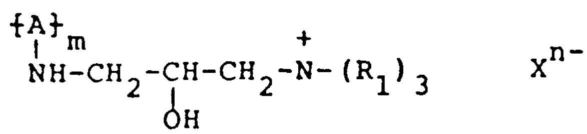

Formula II

in which A represents the monomer unit of a chitosan, m is a whole number between 100 and 1,000, $R_1$ is $C_1$-$C_4$ linear alkyl and $X^{n-}$ is a physiologically acceptable water soluble polyanion where n is at least 10 but not greater than 10,000 and any remaining positive charges are balanced by nonpolymeric anions, wherein said polyanion is gum arabic or carboxymethylcellulose and the proportion of polycation to polyanion $X^{n-}$ is from 1 to 5, to 20 to 1 by weight.

**2.** The salts as recited in claim 1 wherein the alkyl substituent is methyl.

**3.** A process of preparing quaternary ammonium salts of chitosan as claim in claim 1 comprising contacting a compound of the formula:

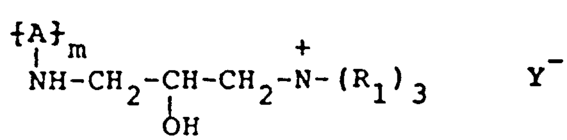

Formula III

in which A represents the monomer unit of a chitosan, m is a whole number between 100 and 1,000, $R_1$ is $C_1$-$C_4$ linear alkyl, and $Y^-$ is a physiologically acceptable water soluble inorganic or monomeric organic counterion, with gum arabic or carboxymethylcellulose.

**4.** The process as recited in claim 3 wherein said contacting occurs in an aqueous solution.

**5.** A pharmaceutical composition having hypocholesterolemic activity comprising a compound of claim 1 in a pharmaceutically acceptable carrier.

**6.** The pharmaceutical composition as recited in claim 5, said composition in solid form, and including sufficient inorganic salt such that said composition is water soluble.

**7.** The method of making the composition as recited in claim 6 comprising dissolving a compound of the formula:

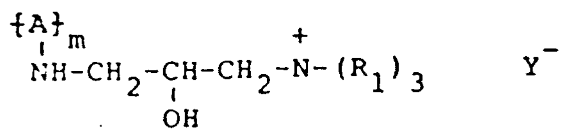

Formula III

in which A represents the monomer unit of a chitosan, m is a whole number between 100 and 1,000, $R_1$ is $C_1$-$C_4$ linear alkyl and $Y^-$ is a physiologically acceptable water soluble inorganic or monomeric organic counterion, and gum arabic or carboxymethylcellulose , in a common protic solvent;

precipitating the thus formed polycation-polyanion salt;

collecting said polycation-polyanion salt; and adding sufficient inorganic salt such that said polycation-polyanion salt is substantially soluble upon mixing in an aqueous solvent.

**8.** The method of making the composition as recited in claim 7 wherein said precipitation occurs by reducing the ionic strength of the solution.

**9.** The method of making the composition as recited in claim 8 wherein said ionic strength is reduced by diluting said solution.

**Claims for the following Contracting States : ES, GR**

**1.** A process for preparing quaternary ammonium salts of chitosan possessing hypocholesterolemic activity of the formula:

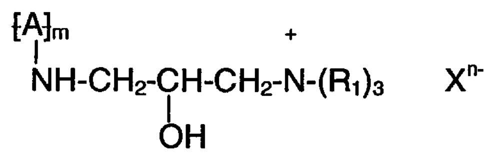

Formula II

in which A represents the monomer unit of a chitosan, m is a whole number between 100 and 1,000, $R_1$ is $C_1$-$C_4$ linear alkyl and $X^{n-}$ is a physiologically acceptable water soluble polyanion where n is at least 10 but not greater than 10,000 and any remaining positive charges are balanced by nonpolymeric anions, wherein said polyanion is gum arabic or carboxymethylcellulose and the proportion of polycation to polyanion $X^{n-}$ is from 1 to 5, to 20 to 1 by weight, which comprising contacting a compound of the formula:

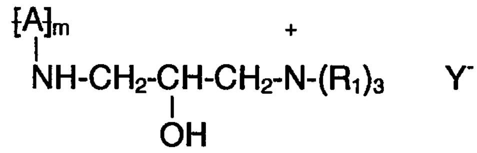

Formula III

wherein A, m and $R^1$ are as previously defined and $Y^-$ is a physiologically acceptable water soluble inorganic or monomeric organic counterion, with gum arabic or carboxymethylcellulose.

2. A process as claimed in claim 1 wherein the alkyl substituent is methyl.

3. The process as recited in claim 1 wherein said contacting occurs in an aqueous solution.

4. The method of making a pharmaceutical composition in solid form, and including sufficient inorganic salt such that said composition is water soluble, which comprises dissolving a compound of the formula:

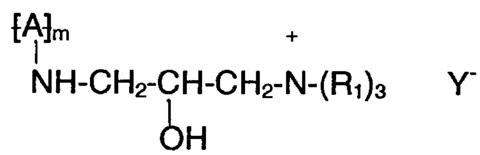

Formula III

wherein A, m, $R^1$ and $Y^-$ are as defined in claim 1, and gum arabic or carboxymethylcellulose in a common protic solvent;

precipitating the thus formed polycation-polyanion salt;

collecting said polycation-polyanion salt; and adding sufficient inorganic salt such that said polycation-polyanion salt is substantially soluble upon mixing in an aqueous solvent.

5. The method of making the composition as recited in claim 4 wherein said precipitation occurs by reducing the ionic strength of the solution.

6. The method of making the composition as recited in claim 5 wherein said ionic strength is reduced by diluting said solution.

**Patentansprüche**

**Patentansprüche für folgende Vertragsstaaten : AT, BE, CH, DE, DK, FR, GB, IT, LI, LU, NL, SE**

1. Quaternäre Ammoniumsalze von Chitosan, die eine hypocholesterolemische Aktivität besitzen, der Formel:

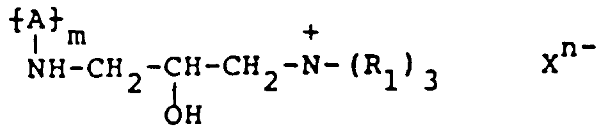

Formel II

worin A die Monomereinheit eines Chitosans repräsentiert, m eine ganze Zahl zwischen 100 und 1.000 ist, $R_1$ $C_1$-$C_4$-lineares Alkyl ist und $X^{n-}$ ein physiologisch verträgliches wasserlösliches Polyanion ist, wobei n mindestens 10, aber nicht größer als 10.000 ist und jegliche verbleibenden positiven Ladungen durch nichtpolymere Anionen ausgeglichen sind, worin das Polyanion Gummiarabicum oder Carboxymethylcellulose ist und das Gewichtsverhältnis von Polykation zu Polyanion $X^{n-}$ von 1 zu 5 bis 20 zu 1 reicht.

**2.** Salze gemäß Anspruch 1, worin der Alkylsubstituent Methyl ist.

**3.** Verfahren zur Herstellung quaternärer Ammoniumsalze von Chitosan gemäß Anspruch 1, umfassend das In-Kontakt-bringen einer Verbindung der Formel:

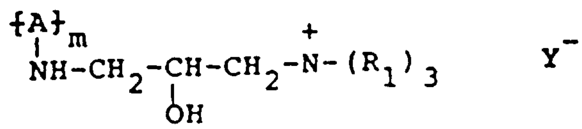

Formel III

worin A die Monomereinheit eines Chitosans repräsentiert, m eine ganze Zahl zwischen 100 und 1.000 ist, $R_1$ $C_1$-$C_4$-lineares Alkyl ist, und $Y^-$ ein physiologisch verträgliches wasserlösliches anorganisches oder monomeres organisches Gegenion ist, mit Gummiarabicum oder Carboxymethylcellulose.

**4.** Verfahren gemäß Anspruch 3, worin das In-Kontakt-bringen in einer wäßrigen Lösung stattfindet.

**5.** Pharmazeutische Zusammensetzung mit hypocholesterolemischer Aktivität, umfassend eine Verbindung von Anspruch 1 in einem pharmazeutisch verträglichen Träger.

**6.** Pharmazeutische Zusammensetzung gemäß Anspruch 5, worin die Zusammensetzung in fester Form vorliegt und ausreichend anorganisches Salz einschließt, so daß die Zusammensetzung wasserlöslich ist.

**7.** Verfahren zur Herstellung der Zusammensetzung gemäß Anspruch 6, umfassend das Lösen einer Verbindung der Formel:

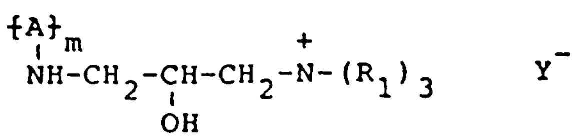

Formel III

worin A die Monomereinheit eines Chitosans repräsentiert, m eine ganze Zahl zwischen 100 und 1.000

ist, $R_1$ $C_1$-$C_4$-lineares Alkyl ist und $Y^-$ ein physiologisch verträgliches wasserlösliches anorganisches oder monomeres organisches Gegenion ist, und Gummiarabicum oder Carboxymethylcellulose, in einem gewöhnlichen protischen Lösungsmittel; das Fällen des so gebildeten Polykationen-Polyanionensalzes; das Sammeln des Polykationen-Polyanionensalzes; und das Zugeben von ausreichend anorganischem Salz, so daß das Polykationen-Polyanionensalz durch Mischen in einem wäßrigen Lösungsmittel im wesentlichen löslich ist.

**8.** Verfahren zur Herstellung der Zusammensetzung gemäß Anspruch 7, worin die Fällung durch Reduzierung der Ionenstärke der Lösung geschieht.

**9.** Verfahren zur Herstellung der Zusammensetzung gemäß Anspruch 8, worin die Ionenstärke durch Verdünnen der Lösung reduziert wird.

**Patentansprüche für folgende Vertragsstaaten : ES, GR**

**1.** Verfahren zur Herstellung quaternärer Ammoniumsalze von Chitosan, die eine hypocholesterolemische Aktivität besitzen, der Formel:

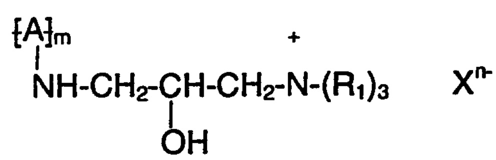

Formel II

worin A die Monomereinheit eines Chitosans repräsentiert, m eine ganze Zahl zwischen 100 und 1.000 ist, $R_1$ $C_1$-$C_4$-lineares Alkyl ist und $X^{n-}$ ein physiologisch verträgliches wasserlösliches Polyanion ist, wobei n mindestens 10, aber nicht größer als 10.000 ist und jegliche verbleibenden positiven Ladungen durch nichtpolymere Anionen ausgeglichen sind, worin das Polyanion Gummiarabicum oder Carboxymethylcellulose ist und das Gewichtsverhältnis von Polykation zu Polyanion $X^{n-}$ von 1 zu 5 bis 20 zu 1 reicht, umfassend das In-Kontakt-bringen einer Verbindung der Formel:

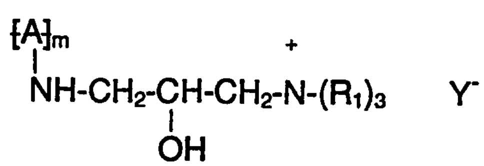

Formel III

worin A, m und $R_1$ wie zuvor definiert sind und $Y^-$ ein physiologisch verträgliches wasserlösliches anorganisches oder monomeres organisches Gegenion ist, mit Gummiarabicum oder Carboxymethylcellulose.

**2.** Verfahren gemäß Anspruch 1, worin der Alkylsubstituent Methyl ist.

**3.** Verfahren gemäß Anspruch 1, worin das In-Kontakt-bringen in einer wäßrigen Lösung stattfindet.

**4.** Verfahren zur Herstellung einer pharmazeutischen Zusammensetzung in fester Form, die ausreichend anorganisches Salz einschließt, so daß die Zusammensetzung wasserlöslich ist, das das Lösen einer

Verbindung der Formel:

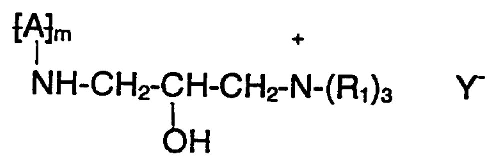

Formel III

worin A, m, $R_1$ und $Y^-$ wie in Anspruch 1 definiert sind, und Gummiarabicum oder Carboxymethylcellulose in einem gewöhnlichen protischen Lösungsmittel; das Fällen des so gebildeten Polykationen-Polyanionensalzes; das Sammeln des Polykationen-Polyanionensalzes; und das Zugeben von ausreichend anorganischem Salz umfaßt, so daß das Polykationen-Polyanionensalz durch Mischen in einem wäßrigen Lösungsmittel im wesentlichen löslich ist.

**5.** Verfahren zur Herstellung der Zusammensetzung gemäß Anspruch 4, worin die Fällung durch Reduzierung der Ionenstärke der Lösung geschieht.

**6.** Verfahren zur Herstellung der Zusammensetzung gemäß Anspruch 5, worin die Ionenstärke durch Verdünnen der Lösung reduziert wird.

**Revendications**
**Revendications pour les Etats contractants suivants : AT, BE, CH, DE, DK, FR, GB, IT, LI, LU, NL, SE**

**1.** Sels d'ammonium quaternaire de chitosane, présentant une activité hypocholestérolémique, de formule :

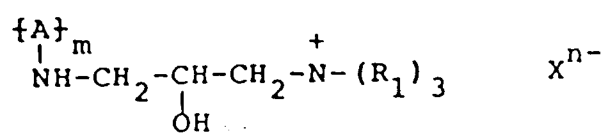

Formule II

dans laquelle A représente le motif monomérique du chitosane, m est un nombre entier de 100 à 1000, $R_1$ représente un groupe alkyle linéaire en $C_1$ à $C_4$ et $X^{n-}$ représente un polyanion hydrosoluble physiologiquement acceptable dans lequel n est au moins égal à 10 mais non supérieur à 10 000 et toutes les charges positives restantes sont équilibrées par des anions non polymériques, ledit polyanion consistant en gomme arabique ou carboxyméthycellulose, et le rapport de polycation au polyanion $X^{n-}$ étant compris dans l'intervalle d'une valeur de 1 à 5 à une valeur de 20 à 1, en poids.

**2.** Sels suivant la revendication 1, dans lesquels le substituant alkyle est un substituant méthyle.

**3.** Procédé de préparation de sels d'ammonium quaternaire de chitosane suivant la revendication 1, comprenant la mise en contact d'un composé de formule :

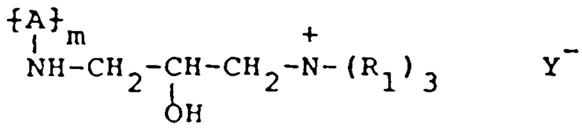

Formule III

dans laquelle A représente le motif monomérique du chitosane, m est un nombre entier de 100 à 1000, $R_1$ représente un groupe alkyle linéaire en $C_1$ à $C_4$ et $Y^-$ représente un ion complémentaire inorganique ou organique monomérique hydrosoluble physiologiquement acceptable, avec la gomme arabique ou la carboxyméthylcellulose.

**4.** Procédé suivant la revendication 3, dans lequel la mise en contact se produit dans une solution aqueuse.

**5.** Composition pharmaceutique douée d'activité hypocholestérolémique, comprenant un composé suivant la revendication 1, dans un véhicule pharmaceutiquement acceptable.

**6.** Composition pharmaceutique suivant la revendication 5, ladite composition étant sous forme solide et comprenant une quantité d'un sel inorganique suffisante pour que ladite composition soit hydrosoluble.

**7.** Procédé de préparation de la composition suivant la revendication 6, comprenant la dissolution du composé de formule :

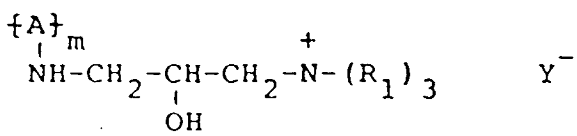

Formule III

dans laquelle A représente un motif monomérique du chitosane, m est un nombre entier de 100 à 1000, $R_1$ représente un groupe alkyle linéaire en $C_1$ à $C_4$ et $Y^-$ représente un ion complémentaire inorganique ou organique monomérique hydrosoluble physiologiquement acceptable, et de la gomme arabique ou de la carboxyméthylcellulose dans un solvant protique commun ;

la précipitation du sel de polycation-polyanion ainsi formé ;

la séparation dudit sel de polycation-polyanion ; et l'addition d'une quantité d'un sel inorganique suffisante pour que ledit sel de polycation-polyanion soit soluble de manière effective lors de son mélange à un solvant aqueux.

**8.** Procédé de préparation de la composition suivant la revendication 7, dans lequel la précipitation se produit par diminution de la force ionique de la solution.

**9.** Procédé de préparation de la composition suivant la revendication 8, dans lequel la force ionique est réduite par dilution de la solution.

**Revendications pour les Etats contractants suivants : ES, GR**

**1.** Procédé de préparation de sels d'ammonium quaternaire de chitosane, présentant une activité hypocholestérolémique, de formule :

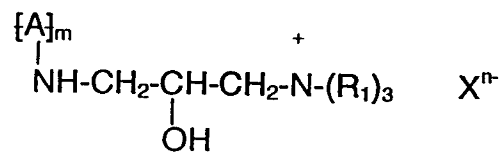

Formule II

dans laquelle A représente le motif monomérique du chitosane, m est un nombre entier de 100 à 1000, $R_1$ représente un groupe alkyle linéaire en $C_1$ à $C_4$ et $X^{n-}$ représente un polyanion hydrosoluble physiologiquement acceptable dans lequel n est au moins égal à 10 mais non supérieur à 10 000 et toutes les charges positives restantes sont équilibrées par des anions non polymériques, ledit polyanion consistant en gomme arabique ou carboxyméthylcellulose et le rapport du polycation au polyanion $X^{n-}$ étant compris dans l'intervalle d'une valeur de 1 à 5 à une valeur de 20 à 1 en poids, qui comprend la mise en contact d'un composé de formule :

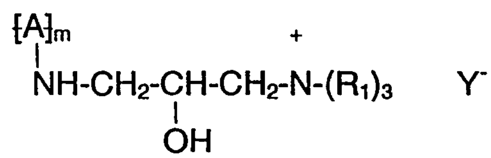

Formule III

dans laquelle A, m et $R^1$ répondent aux définitions précitées et $Y^-$ représente un ion complémentaire inorganique ou organique monomérique hydrosoluble physiologiquement acceptable, avec de la gomme arabique ou de la carboxyméthylcellulose.

**2.** Procédé suivant la revendication 1, dans lequel le substituant alkyle est un substituant méthyle.

**3.** Procédé suivant la revendication 1, dans lequel la mise en contact se produit dans une solution aqueuse.

**4.** Procédé de préparation d'une composition pharmaceutique sous forme solide, comprenant une quantité d'un sel inorganique suffisante pour que ladite composition soit hydrosoluble, qui comprend la dissolution d'un composé de formule :

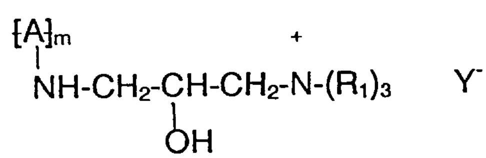

Formule III

dans laquelle A, m, $R^1$ et $Y^-$ répondent aux définitions suivant la revendication 1, et de gomme arabique ou de carboxyméthylcellulose dans un solvant protique commun ;

la précipitation du sel de polycation-polyanion ainsi formé ;

la séparation dudit sel de polycation-polyanion ; et l'addition d'une quantité d'un sel inorganique suffisante pour que ledit sel de polycation-polyanion soit soluble de manière effective lors de son mélange à un solvant aqueux.

**5.** Procédé de préparation de la composition suivant la revendication 4, dans lequel la précipitation se produit par diminution de la force ionique de la solution.

**6.** Procédé de préparation de la composition suivant la revendication 5, dans lequel la force ionique est réduite par dilution de la solution.